# EUROPEAN PATENT APPLICATION

(11) **EP 0 982 033 A2**
(43) Date of publication of application: **01.03.2000**
(21) Application number: 99114058.3
(22) Date of filing: 11.12.1996
(51) Int. Cl.: A61K 31/70

(54) **Use for fructose-1,6-diphosphate (FDP) in the manufacture of medicaments**

(30) Priority: 08.05.1996 US 646600; 09.05.1996 US 647206; 30.08.1996 US 705773; 30.08.1996 US 705789
(62) Divisional of application: 96309024.6
(71) Applicant: Cypros Pharmaceutical Corporation, Carlsbad, California 92008 (US)
(72) Inventor: Fox, Anthony W., Rancho LaCosta, CA 92009 (US); Marangos, Paul J., Encinitas, CA 92024 (US)
(74) Representative: Woods, Geoffrey Corlett

(57) **Abstract**

Use of fructose-1,6-diphosphate or a pharmacologically acceptable salt thereof in the manufacture of a medicament for use in a method for treating a human patient preparing to undergo circulatory bypass of the heart during surgery or for use in the emergency treatment of a patient.

## Description

### BACKGROUND OF THE INVENTION

*Glycolysis* is a fundamental biological process which is essential to the generation and use of energy by cells. It is discussed in nearly any textbook on biochemistry, physiology, or cell biology; see, e.g., any edition of Stryer's or Lehninger's *Biochemistry*, Guyton's *Medical Physiology*, or Alberts et al, *Molecular Biology of the Cell*.

Fructose-1,6-diphosphate is a naturally occurring sugar-phosphate molecule, which is created and then quickly consumed as an intermediate during glycolysis. Some scientists refer to FDP as fructose-1,6-biphosphate, or fructose-1,6-bisphosphate. As a short-lived intermediate, it normally is present in cells only at relatively low concentrations.

The 1,6-isomer of fructose diphosphate, which contains phosphate groups bonded to the #1 and #6 carbon atoms of the fructose molecule, is the only isomer of interest herein, and it is abbreviated herein as FDP. Other isomers (such as fructose-2,6-diphosphate) are not relevant to this invention, and are excluded from any references to FDP or fructose diphosphate.

Over the past 20 years, dozens of articles published by scientists and physicians have suggested that FDP might be useful in preventing ischemic damage to various types of organs. Such articles include, for example, Markov et al 1980, 1986, and 1987; Gregory et al 1990; Trimarchi et al 1993 and 1994; and Sano et al 1995. Relevant patents include US patents 4,546,095 (Markov 1985), 4,703,040 (Markov 1987), and 4,757,052 (Markov 1988). These are just a few examples; there are many others.

In addition, various patents describe methods for synthesizing or purifying FDP. The synthesis methods usually involve microbes which will convert glucose into FDP, if provided with glucose and a phosphate donor under proper fermentation conditions; these include Chinese patent 1,089,654 A (Yin, 1994) and US patents 4,530,902 (Perri et al 1985) and 5,094,947 (Nakajima et al, 1991). The purification patents usually involve ion exchange chromatography; see US patents 4,575,549 (Diana et al, 1986; apparently equivalent to German patent DE 3,446,927) and 5,434,255 (Katayama et al, 1995; apparently equivalent to Japanese patent JP 05271269 A2) as well as Chinese patents 1,089 615 A (Ying et al 1994) and 1,089,616 A (Ouyang et al 1994).

Despite this prior art, which stretches back nearly twenty years, a high degree of skepticism and reluctance still exists among physicians and surgeons. With a few very limited exceptions, discussed below, FDP simply is not used by physicians or surgeons for medical purposes.

The failure or refusal of doctors to use FDP on patients who are suffering from ischemia (including, in many cases, patients who are dying of massive heart attacks, cardiac arrest, strokes, or blood loss, and who desperately need any possible energy supply) is believed to be due to a number of factors, including:
(1) FDP is a diphosphate with a strong negative charge, and it is generally assumed that it cannot enter cells in substantial quantities. Since energy metabolism and glycolysis occur inside cells, it is assumed that FDP will not get to the relevant sites in sufficient quantities to do any good.
(2) It is also assumed that FDP cannot readily penetrate the blood-brain barrier, so it will be of no utility in protecting the brain. Since the brain is much more vulnerable to rapid and permanent damage from ischemia or hypoxia than any other organ, this leads to a general belief that any benefits that might be provided by protecting the heart or other internal organs against ischemic damage would be minor, and will not address or help solve the critical problem of preventing permanent brain damage, which outranks all other potential problems.
(3) The amount of energy generated during glycolysis (i.e., the splitting of glucose to form two molecules of pyruvate) is only a small fraction of the energy generated by the subsequent aerobic oxidation of pyruvic acid into its ultimate products, carbon dioxide and water. Under conditions of tissue ischemia or hypoxia, where an oxygen deficit blocks aerobic conversion of pyruvate, and generates undesired lactic acid instead, it is assumed that FDP infusion would be insufficient to supplement ATP levels to a degree that can significantly aid cell survival.
(4) It is also assumed that addition of exogenous FDP leads to an increase in lactic acid concentrations, under oxygen-deficient conditions. This can be harmful, since excess lactic acid can shut down a crucial enzyme called phosphofructokinase.
(5) Drug treatments for ischemic trauma have proven to be very difficult and unsatisfactory, for many reasons. Among other things, it often requires 1 to 3 hours (or more) before a patient can be properly diagnosed in a manner that justifies the use of a specific drug. However, if that much time elapses after the onset of ischemia, before drug delivery begins, it is often too late to intervene effectively because extensive cell death and permanent tissue damage will have already occurred.
(6) Contrary to the articles which reported that FDP may have beneficial effects, a number of other articles have reported that FDP had no beneficial effects in various studies. Examples of these negative articles include Eddy et al 1981, Pasque et al 1984, Tortosa et al 1993, and Angelos et al 1993.
(7) In the past, FDP has proven to be difficult to create in a pure and stable form. It hydrolyzes readily in contact with water, and if a single phosphate group is cleaved off, the remaining molecule is effectively useless to accomplish the goals of FDP. Previous efforts at lyophilization apparently have not proven to be effective at creating pure ans stable versions; by way of illustration, Chinese patent 1,089,616 (Ouyang et al, 1994) claimed a method of high-temperature drying, to avoid the problems that had been encountered in lyophilization efforts. The crucial factors of purity and stability are discussed in more detail below.

For these and other reasons, it appears that very little effort has been directed by the pharmaceutical industry toward actually developing and using FDP as a drug. With a few possible exceptions, FDP simply is not administered to any patients on any sort of routine basis, regardless of how desperate their plight may be following a stroke, cardiac arrest, shooting, stabbing, etc.

The first exception involves a lyophilized preparation sold under the trademark ESAFOSFINA by an Italian company called Biochemica Foscama, described below. Apparently, that compound is sold in a few countries, such as China and Italy; it may be administered to humans in those countries, even though it may not meet the requirements for purity or for sterile manufacturing that apply to drugs in other countries. And second, FDP is occasionally tested in small-scale clinical trials, in countries such as the United States and Great Britain.

Currently, there are only two known preparations of FDP which are commercially available anywhere in the world, other than research reagents that are sold in gram or milligram quantities by specialty chemical companies. One of these preparations is a non-sterile bulk powder, manufactured in Germany by Boehringer Mannheim. This material was purchased by the assignee (Cypros Pharmaceutical Corporation, of Carlsbad, California) and used as the starting reagent for the lyophilized preparation described herein.

The other commercially available FDP formulation is the lyophilized preparation that is manufactured in Italy by Biochemica Foscama, and sold under the trademark ESAFOSFINA. To the best of the Applicant's knowledge and belief, it is manufactured by steps that including the following: (1) pouring a large volume of an aqueous mixture of FDP into a large, flat tray; (2) freezing the mixture and subjecting it to a vacuum, to remove the water, thereby creating a large solidified cake; (3) grinding or milling the large cake into small particles; (4) loading the ground-up particles into small vials; and, (5) sealing the vials.

This process is not well-suited for creating a sterile preparation for injection into humans. For example, a manufacturing process which uses large machinery to handle and manipulate a large cake, pass it through a device which grinds it up into small particles, pass the particles through various routing and funnelling devices in order to load those particles into small vials, and then seal the vials, creates numerous risks which seriously jeopardize the sterility of the resulting final product (which is a phospho-sugar compound that can readily support dangerous microbial growth).

Regrettably, these problems cannot be overcome by a "terminal sterilization" process, such as autoclaving or ionizing radiation of the FDP after it has been loaded into the vials. Such treatments would seriously degrade the chemical quality and purity of the FDP in the vials. Furthermore, it should be recognized that FDP is easily digested and readily used as a nutrient by a wide variety of bacteria; if there is any bacterial contamination in a sealed vial of FDP, the bacteria might well multiply in the vial.

The final product of the previously known lyophilized FDP preparation from also suffers from several apparent shortcomings. It is relatively inhomogeneous, and contains particles of different sizes; some appear to be small glass-like beads, while others appear to be relatively sticky, caramelized agglomerations. When a sample was tested for chemical purity, it violated the purity standards described below. It is also somewhat unstable; while pure FDP is a crystalline white powder, some of the particles in the prior art preparation (especially the larger particles) begin to turn a yellowish-brown color within a few days or weeks, when stored at room temperature, unopened, in the sealed vials.

To the best of the Applicant's knowledge and belief, neither the Boehringer Mannheim company (headquartered in Germany) nor the Biochemica Foscama company (located in Italy) have ever made any effort to obtain permission from the Food and Drug Administration (FDA) in the United States, the Medicines Control Agency (MCA) in Great Britain, or other comparable drug control agencies in other major industrialized nations, to sell their FDP preparations for use on humans. Accordingly, the sale of FDP for injection into humans apparently is prohibited in nearly all countries, even if prescribed by doctors. Prior to the Applicant herein, it appears that no company has taken the time or effort to create a pure and sterile preparation which has sufficient shelf life to be a practical and effective treatment on a commercial scale, and to obtain proper governmental approval for sale and use of FDP in countries that impose strict purity and safety standards on drugs.

### Lyophilization

Lyophilization is the technical name for a process often called "freeze-drying". In this process, an aqueous mixture or suspension is frozen into a solid, then it is subjected to a vacuum for a substantial period of time (usually a full day or more). The vacuum causes the water molecules to "sublimate", i.e., to become gaseous and leave the solid, without going through a liquid state. This is comparable to the evaporation of carbon dioxide from "dry ice", but it is much slower, and it requires high levels of vacuum, which can only be generated in a special chamber that can generate and sustain both (1) very cold freezing temperatures, which are much lower than the typical temperatures of conventional household freezers, and (2) very high levels of vacuum, measured in millitorr (thousandths of a torr; typical atmospheric pressure is 760 torr, or 760 millimeters of mercury column in a U-shaped device called a manometer).

Because of the extremely high requirements for sterility and quality control, lyophilization of pharmaceuticals is a very expensive process. It requires a lot of energy to sustain the proper freezing and vacuum conditions in a lyophilization chamber, and each batch of drugs must sit in an expensive chamber, absolutely motionless, for many hours or days. For these and other reasons, any processing improvement that can speed up a lyophilization protocol without compromising the quality or sterility of the product is regarded as an improvement over longer, slower processes.

### Residual Moisture Levels

One of the most important factors in lyophilizing a drug product is the final water content. Most lyophilization protocols include both a "primary drying" stage, which reduces moisture content to a level of about 7% to 10%, by weight, followed by a "secondary drying" stage, which usually reduces the water content to less than 2%. Reducing residual water content to less than about 2% helps keep hydrolysis (or other chemical reactions that might alter the chemical bonds in the drug molecule) to a minimum.

Relevant prior art which discusses residual moisture content includes the following:
(1) U.S. Patent 4,537,883, which states: "Final moisture content of the dried product is generally below 1.0%, although some products, mainly biologicals, may have a final moisture content which could range as high as about 10%. Usually, the improvement in stability of the lyophilizate, compared to the solution, is due to the absence of water in the pharmaceutical composition."
(2) Adams, G. D. J., *Freeze-Drying of Biological Materials, Drying Technology*, 9(4), (1991), pages 891-925, which states: "The prolonged drying stage is called secondary drying, water being removed by desorption. During secondary drying the water content is reduced to 2% or less."
(3) Greiff, D., "Freeze-Drying Cycles," in *International Symposium on Freeze Drying of Biological Products* (published by S. Karger, Basel, Switzerland, 1977, pp. 105-115): "During the drying process, suspensions will contain biologic materials in at least three states: (1) dried materials containing low contents of residual moisture (3-5%), (2) dried material containing less than 3% residual moisture, and (3) non-dried, frozen material."
(4) A section entitled, "Formulation and Process Guidelines," by Michael J. Pikal and Steven L. Nail, in the printed course notes from *Lyophilization: A Short Course*, sponsored by Parenteral Drug Association, Inc. (March 13-14, 1996), states as follows: "Secondary Drying: . . . The first question is, 'What is the desired level of residual water?' This issue should be addressed during formulation development studies. In many cases, the lower the residual moisture, the more stable is the product. However, there may not be a significant difference in stability between zero water, and some moderate moisture level, such as 1% . . . An optimum level of water which is relatively high (5%) would be difficult, and formulation approaches should be explored in an attempt to circumvent this requirement."
(5) *Guidelines for the Determination of Residual Moisture in Dried Biological Products*, Docket No. 89D-0140, January 1990, issued by CBER (the Center for Biologicals Evaluation and Research), which is part of the U.S. Food and Drug Administration: "Residual moisture has been the term used to describe the low level of surface water, usually from less than 1% to 5%, remaining in a freeze-dried vaccine or other biological product after the bulk of the aqueous solvent has been removed during the freeze-drying (vacuum sublimation) process."

The Applicant is not aware of any commercially available lyophilized drug with a residual moisture greater than 10%. Accordingly, the Applicant's discovery that FDP can be "partially lyophilized", allowing residual water contents to remain at least 10%, and up to about 25%, while retaining excellent stability, would have been very surprising for any type of drug. It was even more surprising for fructose diphosphate, since its two phosphate bonds were expected to be quite vulnerable to hydrolysis.

### Chemical Purity and Stability Standards

A common and widely used standard for chemical purity, for drugs intended for human use, is as follows: a drug preparation must not contain more than 5% total impurities, and it must not contain more than 1% of any single impurity (as used herein, "impurity" excludes water or other diluents or carriers, and deliberate additives). This standard has been applied and enforced on all clinical trials of FDP by the Applicant, by the appropriate governmental agencies; this included the Food and Drug Administration (FDA) in the U.S.A., and the Medicines Control Agency (MCA) in Great Britain.

FDP is chemically degraded when any carbon-oxygen or oxygen-phosphorous bond is broken or altered. This includes the loss of either phosphate group from the molecule, which is a major problem, since the phosphate bonds are highly susceptible to hydrolysis (water-induced cleavage). If either phosphate group leaves, the resulting fructose monophosphate is worthless. Fructose monophosphate can be distinguished from FDP using high performance liquid chromatography (HPLC).

Chemical degradation also includes a process called "caramelization," in which separate molecules become bonded to each other, to form agglomerated molecules. This is another major problem in any sugar-containing solution.

However, FDP may ionize, or be converted into a salt, without being degraded, and without losing its activity in glycolysis. Ionization and salt formation are not regarded as chemical degradation. The two phosphate groups are highly acidic, and they are most stable in a salt form in any aqueous solution. It should also be noted that non-toxic solvents used in the manufacturing process (such as water and tertiary butyl alcohol) are not regarded as impurities.

In FDP, the 1% limit for any single impurity is crucial. Because of hydrolysis (i.e., cleavage of either phosphate group, to convert the FDP into fructose monophosphate), the 1% single impurity limit is usually violated long before the 5% limit for total impurities is reached.

This factor strongly supports the assertion that partial lyophilization of FDP, which leaves in a water content higher than 10% to give a stable preparation, would not be obvious to anyone with ordinary skill in the art. Since hydrolysis of the phosphate bonds of FDP is the crucial problem that must be overcome to provide a preparation with adequate stability, anyone with ordinary skill in lyophilization chemistry would normally assume that the water content must be reduced as low as possible, even if extreme measures are required, to reduce the risk of hydrolysis. The result discovered by the Applicant points in the exact opposite direction from what was expected.

Another important issue in FDP lyophilization is chemical stability, which is required to sustain purity after reasonable periods of storage, such as a month or more. The goal is to create FDP preparations that have sufficient stability and shelf life to be medically useful, without suffering serious chemical degradation, when stored and handled in sealed vials without refrigeration for prolonged periods. The ability of a preparation to reach or pass this goal can be quantified by using "benchmark" tests, so that compliance or noncompliance of any given batch (or manufacturing process) can be evaluated by impartial standards.

A one-month stability test is used herein as a benchmark standard, for analytical and comparative purposes. If a sterile sealed preparation can be stored at room temperature for at least a month and still meet the 1% single, 5% total impurity standards, it is deemed to have sufficient purity and stability for practical, widespread use. The FDP preparations disclosed herein surpass these purity and stability standards by a wide margin; for example, partially lyophilized samples that were opened and analyzed shortly before this application was filed contained less than 1% total impurities, after more than a year of storage in their sealed vials at room temperature.

Accordingly, one object of this invention is to disclose a method of partially lyophilizing FDP, to create a sterile sealed product that contains at least 10% and up to about 25% residual water content, by weight.

Another object of this invention is to disclose that partially lyophilized FDP which contains 10% to 25% residual water, by weight, is chemically stable and has good shelf life when stored for months at room temperature without refrigeration.

Another object of this invention is to disclose that partially lyophilized FDP preparations with a residual water content between about 12% and about 16% are especially preferred, since they can be created in a more consistent, reproducible, and reliable manner than preparations containing less than 12% or more than 16% water.

Another object of this invention is to disclose that secondary vaporizing agents, such as tertiary butyl alcohol, can be used during the lyophilization of FDP to provide improved final products.

These inventions and disclosures enable the practical, effective, and economic use of FDP for a number of important medical purposes, including (1) emergency medical treatment of patients suffering major medical crises, such as a heart attack, shooting, stabbing, suffocation, loss of blood due to accident or trauma, etc.; and (2) pretreatment of patients who must undergo surgery that requires cardiopulmonary bypass, such as coronary artery bypass grafting, or heart valve replacement or repair.

These and other objects of the invention will become more apparent through the following summary, drawings, and description of the preferred embodiments.

### SUMMARY OF THE INVENTION

A practical and economic method is disclosed for preparing a partially lyophilized (freeze-dried) powder or solidified cake containing fructose-1,6-diphosphate (FDP), a naturally-occurring chemical which is an intermediate in glycolysis. Preferred procedures leave at least 10%, and up to about 25%, residual water (by weight) in the powder or cake; residual moisture in the range of about 12 to 16%, by weight, is especially preferred. This surprisingly high moisture content does not degrade or limit FDP's stability or shelf life, and it provides for faster, less expensive processing. The methods disclosed herein also allow direct lyophilization inside a vial or other sealed container that will hold the lyophilized FDP during storage, shipping and handling. This avoids any need for milling, handling, or other treatment under conditions that might endanger its sterility. This stable formulation allows for improved medical uses of FDP, such as pre-treatment of patients being prepared for cardiopulmonary bypass during surgery. An injection kit is also disclosed, for pre-diagnostic emergency treatment of victims suffering major medical crises, such as heart attacks, auto accidents, shootings, stabbings, and near-suffocation.

The present invention provides a method of preparing chemically stable partially lyophilized fructose-1,6-diphosphate or a pharmacologically acceptable salt thereof, which method comprises the following steps:
(a) placing a quantity of a sterile aqueous solution containing fructose-1,6-diphosphate or a pharmacologically acceptable salt thereof in an open vial that can be sealed in a sterile manner;
(b) lyophilizing the aqueous solution in the vial, under suitable freezing and vacuum conditions in a lyophilization chamber, until a sufficient quantity of water has been removed to generate solidified fructose-1,6-diphosphate or salt thereof which comprises from 10% to 25% residual moisture content, by weight; and,
(c) sealing the vial in a watertight manner;
   wherein the steps are carried out under sterile conditions, thereby generating a sterile preparation of solidified fructose-1,6-diphosphate or a pharmacologically acceptable salt thereof which is sufficiently chemically stable so that, after one month of storage in the vial at room temperature, it contains less than 5 percent total impurities, by weight and contains less than 1 percent of any single impurity, by weight.

The invention further provides a sterile, solidified formulation of fructose-1,6-diphosphate or a pharmacologically acceptable salt thereof having a water content from 10 to 25% by weight which is free of any viruses or bacteria, contains less than 5 percent total impurities, by weight, and less than 1 percent of any single impurity, by weight, and which composition is suitable for mixing with an aqueous solution to generate an aqueous pharmaceutical preparation of fructose-1,6-diphosphate or salt thereof suitable for injection into humans.

In a further embodiment there is provided a dosage formulation comprising the sterile solidifed formulation of fuctose-1,6-diphosphate of the invention and a vial with a watertight sealing device, wherein the vial and the watertight sealing device enclose the solidified formulation of fructose-1,6-diphosphate or salt thereof and maintain its sterility.

The invention also provides a fructose-1,6-diphosphate injection kit comprising:
(a) at least one first sealed enclosure device which contains a sterile, solidified formulation of fructose-1,6-diphosphate or salt thereof according to the invention, in a quantity which is therapeutically effective in treating a human patient suffering from an ischemic or hypoxic medical crisis;
(b) at least one second sealed enclosure device which contains a sterile aqueous solution, wherein means are provided for mixing the fructose-1,6-diphosphate or salt thereof with the aqueous solution, to constitute an injectable aqueous mixture of fructose-1,6-diphosphate or salt thereof without sacrificing sterility of the injectable aqueous mixture;
(c) syringe components for intravenous injection, comprising a syringe barrel, a syringe plunger, and a hypodermic needle, enclosed in sterile wrapping; and
(d) means for transferring into the syringe barrel the injectable aqueous-mixture of fructose-1,6-diphosphate or salt thereof, after it has been constituted by mixing the lyophilized form of fructose-1,6-diphosphate or salt thereof with the aqueous solution.

The invention further provides use of fructose-1,6-diphosphate or a pharmacologically acceptable salt thereof in the manufacture of a medicament for use in the emergency treatment of a patient, by intravenous injection of fructose-1,6-diphosphate or salt thereof into a human patient who appears to be suffering from a condition indicating a medical emergency, wherein the condition is selected from:
a. loss of blood;
b. abnormalities in pulse;
c. electrical shock;
d. symptoms of heart ischemia;
e. asphyxiation;
f. loss of consciousness;
g. mental disorientation which does not appear to be related to drugs or alcohol;
h. inability to control motor functions which does not appear to be related to drugs or alcohol; and,
i. apparent haemorrhagic shock,
and wherein the fructose-1,6-diphosphate or salt thereof is intravenously injected into the patient, prior to diagnosis of the patient's condition.

The invention yet further provides use of fructose-1,6-diphosphate or a pharmacologically acceptable salt thereof in the manufacture of a medicament for use in a method for treating a human patient who is preparing to undergo circulatory bypass of the heart during surgery, the method comprising intravenously injecting into the patient a liquid formulation containing fructose-1,6-diphosphate or a salt thereof as a myocardial protective agent, wherein the fructose-1,6-diphosphate or salt thereof is intravenously injected into the patient, prior to commencement of circulatory bypass, under conditions which cause the fructose-1,6-diphosphate or salt thereof to permeate into heart muscle tissue while the heart is still beating, and wherein the fructose-1,6-diphosphate or salt thereof is injected into the patient in a therapeutically effective quantity which has been statistically shown to reduce ischemic heart stress during surgery.

### BRIEF DESCRIPTION OF THE DRAWINGS

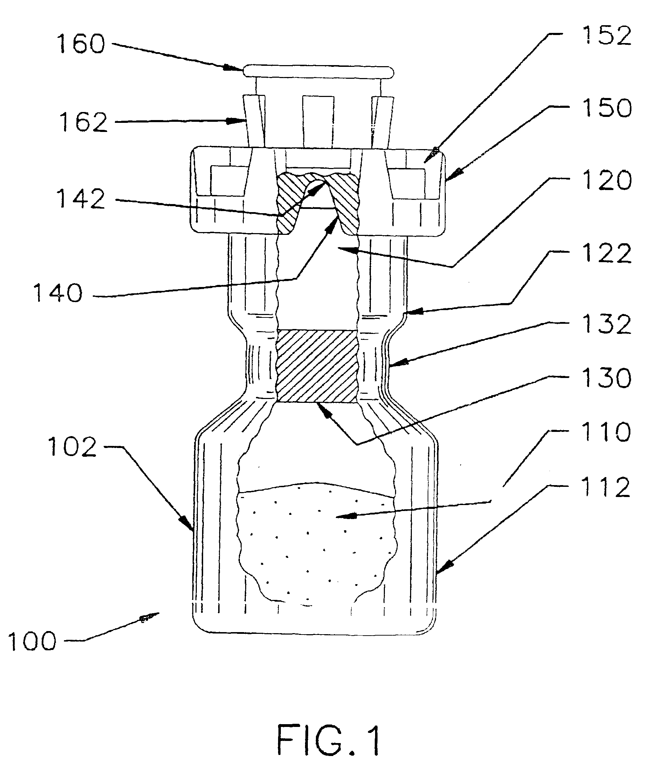

FIGURE 1 is a cutaway side view of a dual-compartment sealed sterile vial containing a partially lyophilized FDP powder or cake in one compartment, and a sterile aqueous solution in a second compartment. The septum that separates the two compartments can be pushed out of its sealed blocking position, to mix the water and the FDP, to reconstitute an aqueous solution of FDP for injection into a human patient.

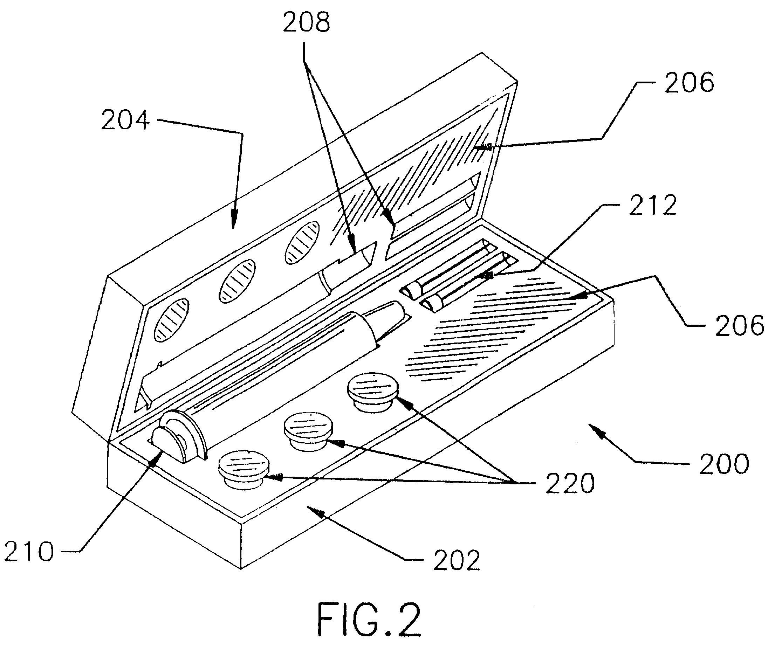

FIGURE 2 depicts a hard-shell case which encloses and protects a hypodermic syringe and needle, and several sealed two-chamber vials which contain both lyophilized FDP and an aqueous solution, to promptly reconstitute the FDP into aqueous solution. This injection kit is designed for emergency use, so that someone who is not a physician, but who is trained to deliver injections (such as ambulance attendants, police, military personnel, nursing home attendants, etc,) can give on-site help in a crisis such as a heart attack, shooting, stabbing, or auto accident.

### DESCRIPTION OF THE PREFERRED EMBODIMENTS

A method is disclosed herein for preparing a partially lyophilized (freeze-dried) powder or solidified cake containing fructose-1,6-diphosphate (FDP), in a powder or cake form that contains residual water in a range of at least 10% and up to about 25%, by weight. This is less than the 26.18% water that is contained in conventional FDP octahydrate crystals, but it is substantially more water than is contained in most conventional forms of fully lyophilized drugs, which usually contain less than 2% residual water. By allowing these high levels of residual water to remain in the partially lyophilized FDP preparations disclosed herein, the energy, equipment, and time requirements for processing are greatly reduced, and the process is rendered practical and economical. However, the partially lyophilized FDP products disclosed herein do not suffer from reduced quality, stability, or sterility.

Results obtained to date also indicate that residual moisture contents in the range of about 12% to about 16% are especially preferred, since this 12 to 16 percent range appears to provide highly consistent and reliable results. Although preparations with good quality have been prepared outside this range, such preparations have been plagued by quality control problems, and they tend to be not as uniformly high in quality. By contrast, preparations with a final moisture content in the range of about 12% to about 16% tend to be more "robust" and reliable, and less vulnerable to undesired quality deviations.

The method disclosed herein produces solidified FDP preparations that are "stable" for at least one month when stored at 25°C in a sealed vial. Terms such as "stable" and "stability" refer to lyophilized preparations that meet or surpass the above-cited widely used purity requirements for human use (5% or less total impurities, and 1% or less of any single impurity, by weight). The storage test period referred to in the claims (one month) is merely a benchmark standard, for convenient and objective analytical and comparative purposes. Many of the FDP preparations disclosed herein have aged for a full year or more, and still surpass the FDA's purity requirements by a comfortable margin.

To be covered by the claims, a "stable" FDP preparation must also remain in good, uniform condition when visually inspected through the glass walls of a vial. It must not suffer obvious levels of caramelization or formation of enlarged or agglomerated particles that are clearly different from the remainder of the lyophilized FDP.

A preferred embodiment of this method of lyophilizing FDP comprises the following steps:
(1) Creating a aqueous solution of FDP, preferably containing FDP in a range of about 10% to about 40% FDP, by weight;
(2) Mixing the aqueous solution of FDP with a second agent which crystalizes at the temperatures of interest and which subsequently sublimates (vaporizes into gas) readily and rapidly at temperatures slightly below the "glass transition temperature" of FDP, which is about -33°C. Due to these physical traits, the second agent creates porosity and permeability in the frozen solid, thereby decreasing the amount of time required for the frozen water to leave the solid during lyophilization, and producing a better final product. One such second agent is tertiary-butyl alcohol, also called t-butanol. Various other agents, such as ethanol, isopropanol, and other organic solvents, have also been used in similar ways.
(3) Loading a predetermined quantity of the sterile mixture into a vial or other small container which is intended to hold the FDP in sterile lyophilized form during storage, shipping, and handling;
(4) Placing the vial and mixture in a lyophilization chamber which can generate and sustain suitable combinations of freezing temperatures and vacuums;
(5) Reducing the temperature of the solution to below the glass transition temperature of FDP, to freeze the mixture;
(6) Subjecting the frozen solid mixture to a primary drying step, in which a predetermined vacuum is sustained for a sufficient time to remove both (a) a majority of the water which is present as ice, and (b) the second agent.
(7) Subjecting the remaining frozen material to a second drying step, which removes some but not all of the so-called "bound" water (water of hydration).

If done properly, these steps will leave behind a powdered or caked residue which contains lyophilized FDP in a high-quality, easily-dissolved form which has good shelf-life, and which also has a residual water content of at least 10% and up to about 25% (even more preferably, to a level of about 12% to 16%), by weight.

As used herein, the term "solidified" is used to refer to powdered, granulated, caked, or other comparable non-liquid forms of FDP which contain less than 25% residual water. For purposes of stability and handling, the important distinction is between liquid mixtures (which are inherently unstable, due to hydrolysis of FDP) and solidified forms, which are stable if prepared properly as described herein. Distinctions between powdered, granulated, or caked forms of FDP are relatively insignificant for the medical uses described herein, since even relatively hard cakes will dissolve fairly rapidly, once water or another suitable liquid has been added to reconstitute FDP into an injectable liquid.

The FDP preparations disclosed herein, having between 10% and 25% residual water, are referred to interchangeably in the text of this application as either "partially lyophilized" or "lyophilized". They are lyophilized, since they have indeed been subjected to a lyophilization process. However, they are only partially lyophilized, when measured by conventional lyophilization standards for pharmaceutical products; several examples of the conventional pharmaceutical standards are cited and quoted in the Background section.

Temperature and vacuum profiles (as a function of time) for carrying out several lyophilization processes are described in Examples 3-7. As indicated therein, exact profiles vary, depending on the initial concentration of FDP in the mixture, and on whether or not TBA is present in the mixture. Examples 3 and 4 indicate early tests, using only 10 or 20% FDP in solution. The procedures in Examples 5-7, using 30 or 40% FDP, are generally preferred. As suggested by the examples, the entire process can be completed in about 2 to 3 days, which is much faster than would be required if full lyophilization were carried out to take the residual water content down to 2% or less.

The exact durations, temperatures, and vacuum levels are not critical, and can be varied if desired, provided that the final lyophilized product meets the relevant quality and stability requirements. For example, if more time is allowed in any particular stage of processing, that stage can be carried out at a lower temperature. However, requirements of more time and lower temperatures both translate into greater expense. Similarly, higher vacuum levels can accelerate the drying process, but may also create other problems, such as "splattering" (a type of boiling in which bubbles burst, causing solid residue to be deposited on the interior walls of the container rather than generating a desirable cake or powder), loss of FDP from a vial, and creation of uneven particle sizes and other inhomogeneities in the final product, and therefore need to be optimized to avoid such problems. Accordingly, the goal is to use the fastest, warmest profile that will consistently and reliably create a stable, uniform, high-quality FDP preparation with a long shelf life.

Another major advantage of the method described herein should also be noted. The lyophilization process disclosed herein can be carried out after a FDP-containing liquid mixture has been loaded into the same vials that will serve as the final sealed and sterile containers for the FDP powder or cake. This eliminates any need for grinding, milling, or other handling or preparative steps that would be required if the powder or cake were to be prepared in bulk (presumably in a large shallow tray) and then ground or milled into particles (or otherwise processed) before being loaded into a small vial. Such processing steps would require very expensive machinery, as well as elaborate and tedious procedures to ensure that sterility is not jeopardized. In-package lyophilization, to remove the water from a liquid mixture that has been loaded into a vial that will serve as its final sealed container, is strongly preferable, and it is enabled in a practical and economic manner by the method disclosed herein.

One of the major benefits of this method is that it substantially reduces costs, and allows for practical and economic commercialization and widespread distribution and sale of partially lyophilized injectable FDP, in a safe and sterile form which is stable for months at room temperature. Lyophilization of other injectable drugs is well known, and methods are taught in the prior art which could, if desired, be directly adapted to creating fully lyophilized FDP, containing a conventional percentage of water as present in most lyophilized drugs (usually about 2% or less, by weight). However, the invention herein is partially based on the discovery that a substantially higher quantity of water can be left in the final powder or cake, without degrading or jeopardizing the stability, safety, or potency of the partially lyophilized FDP, which contains an abnormally high quantity of water (greater than 10%).

Any suitable (i.e., pharmacologically acceptable) salt of FDP can be used, such as a sodium salt, or divalent salts such as calcium or magnesium salts, or mixtures thereof. In general, potassium salts should not be administered intravenously, since an abrupt infusion might interfere with cardiac functioning and certain other cellular functions. The sodium salt is used most commonly, and was used to develop the processing steps described herein. In the quantities that are relevant herein, the amount of sodium contained in this salt will not have any adverse effects on the vast majority of people who are likely to need it. In hospital settings, if sodium causes serious concern in a specific patient, the patient can be treated with a diuretic drug to increase the elimination of sodium, via urine.

It is recognized by the Applicant that other salts are likely to behave differently from the sodium salt, which crystallizes as an octahydrate. Accordingly, other salts (such as calcium, barium, or cyclohexylammonium salts) can also be evaluated for possible use as described herein, if desired.

Isomers other than fructose-1,6-diphosphate cannot be used. Although certain other isomers (including fructose-2,6-diphosphate) occur naturally in cells, they serve other purposes and are not created or consumed as intermediates in the glycolysis pathway.

### Suitable Types of Vials

Partially lyophilized FDP can be stored in any suitable type of sealed container, such as a sealed vial that contains a quantity of FDP suitable for a single dosage for a human adult. The term "vial" is used broadly herein, to refer to any drug-packaging device that is designed and suitable for sealed and sterile storage, shipping, and handling of small (e.g., single-dosage) quantities of drugs. Single-chamber vials (which would contain only the lyophilized FDP, with no water) are well known; a typical single-chamber vial designed for use with intravenous infusion bags (which are carried by any ambulance, and which can enable injection of much larger quantities of liquid drugs than conventional syringes) is illustrated in US patent 4,871,354.

Alternately, two-chamber vials are also known in the art, and can contain both lyophilized FDP and a sterile aqueous solution, to enable immediate reconstitution and injection of an aqueous liquid containing FDP. One example of a conventional two-chamber vial assembly 100 is shown in FIG. 1. This type of vial device is shown in various US patents, including US patent 4,781,354 (Potts 1981). This vial assembly comprises an outer wall 102 which is roughly cylindrical with a flat bottom, and which defines and enclosed a lower chamber 112 (which must be filled first, during the manufacturing process) and an upper chamber 122, separated from each other by a narrower constriction band 132.

This vial structure is conventional and well-known, and the point of novelty of the vial shown in FIG. 1 is the partially lyophilized FDP powder or cake 110 in lower chamber 112. The vial also contains sterile water 120 (preferably in a solution intended for injection that also contains other conventional ingredients such as dextrose, Ringer's lactate, etc.), in upper chamber 122.

The two chambers 112 and 122 are separated from each other by a water-tight partition or "septum" 130, which is a non-permeable disk made of an inert flexible material such as butyl or silicone rubber, which has been force-fitted into the constriction band 132, which holds septum 130 in place until the FDP is needed.

A second non-rigid plug 140, usually made of butyl rubber, is mounted in the neck of the vial, and is secured to the vial by means of a metallic cap 150. This soft, flexible plug 140 allows a sharp tip of a hypodermic or tubing needle to be inserted into upper chamber 122, through a relatively thin upper wall portion 142 of the plug 140. This allows removal of a reconstituted liquid from the vial, so that the liquid can be loaded into a hypodermic syringe or infusion bag, for injection into a patient.

The metallic cap 150 interacts with plunger 160, allowing the plunger to be forced down, through an orifice which occupies the center of the cap 150. Outward-extending locking ears 162 in the sides of plunger 160 interact with accommodating slots 152 in the metallic cap, to lock the plunger in position once it has been pushed down into the cap.

When the FDP is needed for injection into a patient, plunger 160 is depressed. This pushes septum 130 out of position in the constriction band 132, aided by two factors: (a) the inert gas (usually nitrogen or argon) that fills the top of chamber 112 is compressible and allows downward motion of the septum 130 under pressure; and (2) the aqueous liquid which fills the upper chamber is non-compressible, and causes the full force placed on plunger 160 to be pressed against the movable septum 130.

As soon as the septum 130 falls into the lower chamber 112, the dry FDP 110 comes into contact with the aqueous solution 120, and the two are mixed together thoroughly, by shaking the vial. The septum can bounce around inside the lower chamber 112 during this shaking process, and acts as a mechanical agitator to promote full mixing, and to help rapidly break apart the cake, if the dry FDP 110 was in a solidified cake rather than a powder.

A variety of other devices are known in the art, which contain two chambers that are separated from each other in a water-tight manner by a septum. One such device, described in U.S. patent 4,258,845, is sold under the trademark, "Act-O-Vial" by the Upjohn Company (Kalamazoo, MI). Other US patents which also describe other two-chamber devices for drugs include 5,385,546; 5,350,372; 5,336,180; 5,335,773: 4,871,354; and, 4,258,845. There are also several other systems in commercial use, whose patent status is not currently known by the Applicant. These include the "UNIVIAL" system and the "ADD-Vantage" systems, sold by Abbott Laboratories, and the "PIGGYBACK" vial sold by SmithKline Beecham. Nearly any of these devices are suitable for storing a partially lyophilized FDP powder or cake in one chamber, and water in the other chamber.

Another device worth noting is sold under the trademark, "SMART DOSE" by IVAC Medical Systems, San Diego, CA. It is described in US patents 5,398,850 and 5,398,851. Instead of being a self-contained two-chamber device, it is an infusion bag and pump combination which contains a sterile dextrose, Ringer's, or saline solution, and which is designed to be used easily with lyophilized drugs. FDP can be partially lyophilized as disclosed herein, in standard vials (such as the 100 mL vials used in the Examples), and then used in conjunction with the "SMART DOSE" system. This system may provide a highly practical way of administering FDP in certain settings, such as in ambulances.

A sterile, stable lyophilized FDP preparation as disclosed herein can facilitate medical use of FDP, for at least two reasons. First, hydrated forms of FDP generally require refrigeration, even if stored in completely sterile form with no bacteria in them, in order to minimize hydrolysis or other chemical degradation, which is a problem whenever mixtures of FDP dissolved in water must be stored for prolonged periods. Even in settings which have refrigerators, such as hospitals and clinics, refrigeration is a troublesome and expensive storage method. Using lyophilization to eliminate the need for refrigeration (or, for greatly reducing the volumes of any FDP containers that are stored in a refrigerator) provides substantial benefits. And second, hydrated forms of FDP will eventually hydrolyze, even if refrigerated, largely because the two phosphate bonds in FDP are highly vulnerable to hydrolysis (as noted above, the vulnerability of these two bonds to hydrolysis is one of the reasons that the stability of a partially lyophilized FDP product with 10% or more residual water was quite surprising). Minimizing such unwanted chemical degradation, by avoiding aqueous mixtures until immediately before injection, is an important benefit of lyophilization.

### Injection Kits for Use in Medical Emergencies

One or more vials containing lyophilized FDP can be included in an emergency injection kit 200, as shown in FIG. 2. This kit 200 is enclosed in a durable hard-shell case, having a bottom portion 202 and a top portion 204, made of a molded material such as a hard plastic. The external shell can also enclose padding material 206, made of , for example, a lightweight, highly porous foam rubber which has been machined to provide shaped cutaway pockets 208 which will securely hold syringe 210, hypodermic needles 212 (preferably, at least two needles should be provided, in case one gets damaged), and vials 220 containing lyophilized FDP. As many vials as desired can be provided, each vial containing a standard quantity of FDP, such as 5 grams.

The vials 220 can be two-chambered vials, as shown in Fig. 1, for use in police cars, firetrucks, military vehicles, etc., or they can be single-chamber vials, for use in situations where they can be coupled to infusion bags, such as in ambulances, nursing homes, clinics, etc. The syringe and needle can be carried in separated cutaway pockets 208, and the syringe can be sealed inside a sterile plastic wrapper (not shown). Each needle can be carried inside a small rigid plastic shell, to protect it and prevent it from jabbing anything.

Emergency injection kit 200 does not require refrigeration, and the case and padding can protect the vials, syringe, and needles against damage, even if the kit is subjected to rough treatment, as might occur in the trunk of a police car, firetruck, or military vehicle. The kit can also be stored in first aid cabinets, boxes, or closets at locations such as nursing homes, lifeguard stations, and in the homes of people suffering from various diseases, such as heart disease or sickle cell anemia.

The inclusion of lyophilized FDP in this type of emergency injection kit can allow immediate on-site treatment of a patient or victim of a severe medical crisis (such as a heart attack, shooting or stabbing, or auto accident) by anyone who can administer an intravenous injection, such as ambulance attendants, police, firemen, military personnel, nurses, nursing home attendants, emergency room attendants, lifeguards, and relatives of people who suffer from various diseases such as heart disease or sickle cell anemia.

When FDP is administered as quickly as possible to a patient suffering an ischemic or hypoxic crisis, it can substantially reduce cell death and tissue damage, especially in the heart. However, if FDP is mistakenly administered to someone who does not need it and cannot benefit from it, it will not create any known significant adverse effects or pose any known significant risks.

Accordingly, injection of FDP in a crisis situation should not be delayed until a patient can be transported to a doctor or hospital for complete diagnosis and treatment; instead, for maximal effectiveness, injection should occur as soon as possible after an accident or after a patient began to display symptoms of a medical emergency, such as a heart attack.

FDP's combination of valuable traits--protective efficacy in people who need it, combined with very low risk in people who don't need it--render it well-suited for pre-diagnostic use in medical emergencies, using injections that can be administered long before a patient arrives at a hospital for full treatment by a physician. As is well known to physicians, the speed with which proper care is initiated often makes an critical difference in survival rates, in the amount of permanent damage suffered by a patient or victim, and in the personal, family, and social costs that arise after a major medical emergency. This type of emergency pre-diagnostic treatment has never been available before, and it is enabled, for the first time, by the lyophilized form of FDP disclosed herein.

### Medical Conditions Which Warrant Immediate Injection of FDP

Various medical symptoms are listed below, which warrant immediate intravenous injection of FDP by anyone who is qualified to administer an intravenous injection, without waiting for a full diagnosis by a physician. FDP is not recommended or required for minor events; instead, it should be used in situations which indicate a major medical emergency that is severe enough to require an immediate trip to a physician or hospital for full diagnosis and treatment. Such emergencies can include:
1. Substantial loss of blood. This may be due to an accidental injury (such as an auto accident, a fall, mangling by machinery or tools, etc.), an assault such as a shooting or stabbing, or other type of trauma or injury which causes a patient or victim to lose a substantial quantity of blood in a visible, external manner.
2. Abnormalities in pulse which are sufficiently severe to indicate a potential heart attack, cardiac arrest, internal hemorrhage or hemorrhagic shock, or severe allergic reaction. Such abnormalities can include abnormally rapid heartbeat (tachycardia, which in extreme cases becomes fibrillation), abnormally slow heartbeat (bradycardia), arrhythmia (irregularities in the heartbeat), and severe faintness or weakness of the pulse.
3. Electrical shock, which can disrupt proper heart functioning.
4. Symptoms of heart ischemia which may indicate a potential impending heart attack, such as unexplained dizziness or light-headedness, or numbness in one of the limbs (especially in a shoulder or arm).
5. Asphyxiation, such as a near-drowning, suffocation, or carbon monoxide poisoning.
6. Loss of consciousness, apparent hemorrhagic shock, or mental disorientation, unresponsiveness, or inability to control motor functions (such as walking, standing, or sitting straight) which does not appear to be related to use of drugs or alcohol.

If a person loses consciousness, coherence, or motor control with no reason to suspect drug or alcohol abuse and no other clear explanation, it may be due to any of several types of internal crises, such as stroke, internal-hemorrhage, severe allergic reaction to an insect sting or prescription drug, etc. In some cases where these symptoms indicate a medical emergency, injection of FDP may be able to offer extremely useful and beneficial help. In other cases, especially if the crisis is occurring inside the brain (this includes most epilepsy and stroke victims), FDP may provide little if any direct benefit, because of its general inability to penetrate the blood-brain barrier. Yet even in such cases, FDP will pose little or no risk, and it may be able to offer some peripheral benefits even for stroke victims; if useful resources are provided to an adaptive and interactive system that is struggling to cope with a crisis, the system may be able to use those resources to help it sustain homeostatic equilibrium. Accordingly, in cases of unexplained loss of consciousness, coherence, or motor control, an evaluation of FDP's potential to provide helpful protective benefits against its minimal risks and its lack of any known adverse effects, indicates that FDP should be administered promptly, even before a full diagnosis by a physician can be issued.

In summary, FDP may be able to do a great deal of good in many patients suffering from the observable symptoms listed above, while posing no known risk of danger. Accordingly, its rapid pre-diagnostic use is warranted in patients who display the symptoms listed above, if such symptoms indicate that the patient is suffering from a medical crisis that is severe enough to warrant a trip to a hospital.

### Formulations and Dosages For Emergency Use

Preferred dosages for emergency bolus injection from a syringe will generally be in the range of about 50 to about 400 mg/kg (i.e., milligrams of FDP per kilogram of patient body weight). Since most adults weigh between 50 and 100 kg (110 to 220 lb), an emergency injection kit which contains vials holding about 15 grams of FDP can provide dosages in the desired range, with a value of about 300 mg/kg for an adult weighing 50 kg (110 pounds), and about 150 mg/kg for an adult weighing 100 kg (220 pounds).

All of the vials described in the Examples contain 5 grams of FDP in 100 mL molded glass vials. This was used as a standardized quantity during the research described herein. Although it is possible to increase the quantity of FDP in each vial, it is likely that economic and technical factors may lead in the opposite direction (i.e., to smaller quantities of FDP in each vial), for several reasons. First, since vials are arranged in offset rows (in a classical honeycomb pattern) on a shelf in a lyophilization chamber, vials with small diameters make better use of the shelf space inside a chamber, than vials with large diameters. Smaller vials can be packed together more closely, fill up the horizontal area of a shelf better, and leave less empty space between the vials. Second, a relatively shallow liquid in a vial takes less time to lyophilize than a deeper liquid; glass vials are cheap, while chamber time is expensive. And third, vials formed from glass tubing have more consistency in the thickness of their walls and bottoms, than vials formed by molding. This wall uniformity factor reduces potential problems of temperature differentials and product inconsistencies, in products that are lyophilized in vials made from glass tubing. However, tubing vials are usually limited to about 50 mL or less in volume; vials holding 100 mL or more usually need to be made from molded glass.

All of these economic factors point toward smaller vials with smaller quantities of lyophilized FDP. However, preparations containing 5 grams each in molded glass vials have been made with consistently good product quality, in the lab-scale tests described herein, and these results will be evaluated during production scale-up, to determine whether vials holding 5 grams/vial (or possibly even more) can be mass-manufactured without losing product quality.

It is currently believed that nearly any conventional injection liquid (such as a dextrose solution, Ringer's lactate solution, and normal saline solution) can be used as a liquid for reconstituting the lyophilized FDP preparations described herein. Distilled and de-ionized water may even be suitable, since the FDP will normally be in a sodium or other salt form, and can provide sufficient salt to avoid or minimize osmotic disruption after injection.

Alternatively injectable FDP formulations which are reasonably stable can be prepared and stored in fully hydrated form, if desired, if they are properly refrigerated. If the pH is adjusted to less than about 4 or 4.5 using a suitable acid, such as hydrochloric acid, a completely sterile formulation should have a shelf life of several months when refrigerated at 4°C. Eventual degradation of the FDP is characterized by a color change (presumably due to the process called "caramelization", which eventually occurs in nearly all sugars in solution), from clear (which is desirable, and which occurs in fresh preparations) to off-white, through yellow, and eventually to pink.

Preferably a syringe will be pre-loaded with a dosage of fructose-1,6-diphosphate that can significantly reduce ischemic and hypoxic tissue damage, if injected intravenously in a single bolus into an adult human who is suffering a medical crisis. Alternatively, where continuous intravenous infusion is feasible, an FDP formulation can be infused intravenously either as a 10% solution, or after dilution into sterile infusion bags containing 5% dextrose solution, 0.9% saline, Ringer's lactate, or other conventional intravenous fluids (this generally should exclude formulations used for parenteral feeding or blood transfusions, unless approved by a qualified physician). Such infusions preferably should be within a dosage rate of about 1 to about 5 mg/kg per minute, and may follow an initial bolus injection to make more FDP available to ischemic or hypoxic tissue as rapidly as possible.

### Infusion of FDP Prior to Surgery That Requires Cardiopulmonary Bypass

Emergency use is not the only valid use for lyophilized FDP as disclosed herein. Other medical uses of FDP are also rendered substantially more convenient by this partially-lyophilized preparation. For example, it has recently been shown in human clinical trials (designed and funded by the assignee and applicant (Cypros Pharmaceutical Corporation) that if FDP is intravenously infused into a patient who is preparing to undergo coronary artery bypass grafting (CABG) surgery, during roughly a half-hour period before the surgery begins, then the stress and damage which are inflicted on the heart during the surgery will be significantly reduced.

As described in Example 2 pre-treatment with FDP, shortly before cardiopulmonary bypass commenced, significantly reduced unwanted elevations in PAWP (pulmonary anterial wedge pressure) values following surgery. This is important, since elevated PAWP values are present in nearly all manifestations of heart distress. It clearly showed that FDP pretreatment, before bypass surgery, significantly improved the mechanical (hemodynamic) pumping capabilities of the hearts of treated patients after surgery.

These clinical trials also showed a positive correlation between reductions of CK (creatin kinase) blood concentrations, and reductions of PAWP increases. In most cases, the patients who had elevated CK levels in their blood were the same patients who had elevated PAWP values. FDP pre-treatment was able to reduce both damage indicators, compared to untreated control patients.

The ability of FDP pre-treatment to reduce both the biochemical and mechanical indicators of heart damage, simultaneously and with a clear correlation between both sets of indicators, provides strong evidence that this method offers important and measurable benefits in protecting heart cells and heart muscle against ischemic damage during coronary artery bypass grafting surgery.

It is believed that pre-treatment with FDP, shortly before cardiopulmonary bypass begins, can probably help reduce ischemic heart damage in nearly any type of surgery that requires a period of circulatory bypass of the heart and lungs. This includes two types of surgical operations that are common: (1) heart valve replacement, and (2) certain types of surgery to remove or ablate (using microwave radiation or other forms of treatment) segments of heart tissue that are contributing to cardiac arrhythmias. In addition, several new types of cardiac surgery or other treatment are being developed today, which involve or generate various types of temporary cardiac ischemia. One example involves the use of a laser beam to blast relatively small holes in the interior of the left ventricular wall, so that the muscle tissue in the ventricular wall will have greater contact with and access to oxygenated blood that is passing through the left ventricular chamber. It is believed that any such surgical or other treatment procedure which generates any significant level of ischemia in heart muscle tissue can and probably should be preceded by FDP treatment, before the ischemic insult and stress begin, to reduce the risk of ischemic damage to or stress in the affected heart muscle tissue.

Accordingly, this invention teaches the intravenous administration of FDP to patients who are preparing to undergo any form of surgery which involves cardiopulmonary circulatory bypass. The infusion of FDP should begin at an appropriate time shortly before bypass begins, such as about 10 to about 60 minutes before bypass begins. A therapeutically effective dosage, such as about 100 to about 500 milligrams of FDP per kilogram of patient's body weight, should be infused into the patient over a span of at least about 10 minutes, rather than in a single bolus injection.

### EXAMPLES

### EXAMPLE 1: ABILITY OF FDP TO BOOST INTRACELLULAR ATP LEVELS

In order to be effective in boosting glycolysis and generating ATP, exogenous FDP must be able to permeate or be transported into cell interiors, because that is where glycolysis occurs. Since FDP, with its two phosphate groups, carries a strong negative charge, it is widely presumed to suffer from low permeation rates into cells. A large part of the general skepticism among doctors about the cytoprotective capabilities of FDP apparently centers on this presumed limitation.

To evaluate this factor, human astrocytoma cells which had been grown to confluence were incubated for 1 hour with 1 micromolar of tritium-labelled (³H) adenosine (purchased from Morovek Biochemicals, Inc., Brea, California). Any radiolabelled adenosine which remained in the extra-cellular culture medium was washed out after 1 hour.

The cells were then divided into 2 aliquots. One aliquot was incubated for 6 hours with 3.5 mM FDP, while the other aliquot (containing the control population of cells) was incubated identically, but with no FDP. This allowed cells from a single, identically-treated, same-day population to be used as control cells. This approach was used because when a population of cells is supplied with radio-labelled adenosine, it will generate substantially different radiolabelled ATP readings on different days, depending on various factors such as (1) the status and activity levels of the cells on a specific day, (2) variances between labelling intensity in different batches of radiolabelled reagents, and (3) variances in calibration and settings of the analytical equipment. Because of these differences, count-per-minute data tend to be highly variable between different days, while percentage increases are much more consistent and reliable if a single batch of cells is divided into control and treatment aliquots and then treated and tested on a single day using the same reagents and equipment for both aliquots.

After a 6-hour incubation with or without FDP, both aliquots of cells were killed and extracted with 0.4 M perchloric acid and neutralized with an alanine-freon mixture. ATP concentrations were analyzed using polyethylene imine-cellulose thin layer chromatography. Radioactivity was analyzed in a Beckman liquid scintillation counter.

The results showed that under normoxic conditions, the addition of FDP to the extra-cellular liquid roughly doubled the quantity of radiolabelled adenosine converted into ATP; treated cell values averaged 198.7 percent of untreated cell values, with a standard deviation of 17%. Under anaerobic conditions, the boosting effect of extra-cellular FDP on intracellular ATP was even stronger; treated cell values were 243 ± 30%. Since the conversion of adenosine to ATP only occurs inside cells, this provides direct evidence that FDP enters mammalian cells in sufficient quantities to substantially increase ATP levels inside the cells.

### EXAMPLE 2: SAFETY OF FDP IN COMPROMISED PATIENT POPULATIONS

Tests were carried out to evaluate the benefits of FDP in reducing ischemic heart damage in patients who were undergoing circulatory bypass of the heart during coronary artery bypass grafting (CABG) surgery. In these tests, coronary bypass patients were pre-treated with an intravenous infusion of FDP at a dosage of 250 mg of FDP per kilogram of patient body weight, beginning about 10 to 30 minutes before circulatory bypass began. This gave the FDP sufficient time to permeate into the heart tissue before it was subjected to ischemic conditions under cardioplegia.

The results indicated that pre-bypass injection of FDP significantly reduced the level of ischemic damage that was inflicted on treated patients, compared to control populations that did not receive any FDP, as measured by both biochemical and mechanical means. On a biochemical level, FDP significantly reduced the amount of an enzyme called creatine kinase (CK) which was released into blood by cells. Since CK is a very large intracellular protein that cannot be transported across intact cell membranes, this enzyme is commonly used as an indicator of the extent of heart cell death and permanent tissue damage in cardiac patients.

On a mechanical level, pre-bypass treatment with FDP also substantially reduced a hemodynamic abnormality which involves an increase in "pulmonary artery wedge pressure" (PAWP). An increase in PAWP values indicates that a patient's heart has suffered some sort of damage or is struggling with some form of abnormal stress. Nearly all cardiac surgery patients undergo an increase in PAWP values in the hours or days after surgery. If a drug can reduce the undesired increases in PAWP values, it indicates that the drug apparently helped reduce the severity of the stress that was imposed on the heart by the surgery.

There was also a correlation between the biochemical and hemodynamic/mechanical benefits of pre-bypass treatment using FDP. The ability of FDP to reduce the indicators of stress and damage in the heart, using two independent measurements, confirm that FDP can truly benefit the heart and reduce heart stress and damage during circulatory bypass when the heart muscle must undergo a period of ischemia.

These tests were carried out on 20 patients (10 received FDP, and 10 received placebos for control comparisons) under the supervision of a qualified cardiac anesthesiologist. These tests were initiated and sponsored by Cypros Pharmaceutical Corporation (the assignee and applicant herein), which obtained approval from the Food and Drug Administration (FDA, in the U.S.A.) and the Medicines Control Agency (MCA, in Great Britain) prior to carrying out these Phase II human clinical trials. Phase I trial requirements (to establish baseline values for FDP using tests on healthy volunteers) were waived by both the FDA and the MCA, since fructose diphosphate is a naturally occurring biochemical that occurs only as a short-lived intermediate which is quickly consumed during glycolysis.

These tests were carried out using placebo-controlled, double-blind randomized design, and were performed in accordance with all guidelines for Good Clinical Practice (GCP) and the Declaration of Helsinki principles. To the best of the Applicant's knowledge and belief, this was the first such clinical trial ever conducted which tested FDP as a protective agent in this manner or for this use, and there is no other comparable agent being used for this purpose anywhere in the world.

During these tests, there was no indication whatsoever of any adverse effects of FDP on any of the patients, even though all of the patients who were tested were medically compromised and in poor health. A number had already suffered heart attacks, and all were suffering from coronary artery occlusions that were severe enough to require open-chest surgery, rather than balloon angioplasty, drug treatment, or other less-invasive procedures.

Furthermore, since FDP is a short-lived intermediate which is quickly consumed by glycolysis, there is every reason to believe that FDP poses no significant risks, or only very low and minimal risks of any significant adverse effects, in patients who may not need it.

To the best of the Applicant's knowledge and belief, these are the first clinical data ever reported on FDP which were gathered in tests that were carried out under the full auspices of "Good Clinical Practices" in the United States or Great Britain.

### EXAMPLE 3: LYOPHILIZATION OF 10% FDP SOLUTION

After realizing that FDP's potential as a useful drug has been ignored and neglected by pharmaceutical manufacturers, the Applicant, Cypros Pharmaceutical Corporation, commenced a program of developing a suitable lyophilization procedure, to generate a stable and sterile preparation that can be stored for weeks or months and then mixed with water and injected into humans.

This preparation began with a non-sterile bulk powder, manufactured by Boehringer-Mannheim, in Germany. This powder is purchased as a tri-sodium salt. The crystalline form is an octahydrate, with eight molecules of water associated with each molecule of FDP. Calculated water comprises 26.18%, by weight, of this octahydrate salt. Although the concept of "water of hydration" (i.e., the water that remains bound to the FDP when it is in crystalline form) becomes irrelevant after FDP is dissolved in water, a reduction of the water content below 26%, in a lyophilized preparation, requires removal of at least a portion of the water that would remain bound if the FDP were in a normal crystallized form.

During the freezing process, if the FDP forms a glass (i.e., a solid that does not have a regular molecular lattice structure), there will be "sorbed" or "bound" water associated with the non-crystalline material. Such water can be removed by lyophilization. If the product (or some portion thereof) crystallizes during freezing, the product may be an octahydrate or some other hydrated crystalline form, and it is very difficult to remove such water by conventional lyophilization techniques, without melting or altering the crystals.

The bulk powder described above was thoroughly dissolved in water, at varying concentrations starting at about 10% by weight, and ranging up to about 40% by weight. The liquid was pumped through a Gelman "Acrodisc 50" filter with a pore size of 0.2 microns. This sub-micron filtering serves as a sterilization step which removes any bacteria or viral particles. No excipients were added, and the pH was not adjusted.

Filtered sterile solutions were filled into 100 mL molded glass vials. Varying amounts of liquid were loaded into each vial, depending on the concentration of the liquid, to create final quantities of 5 grams of FDP (octahydrate salt) in each vial throughout the tests described herein.

Permeable lyophilization stoppers were placed on the vials, and the vials were loaded onto the shelves in a lyophilization chamber (manufactured by The Virtis Company, Inc., Gardiner, NY) at 13°C. Temperature probes were placed in four interior vials on each shelf.

All lyophilizations described in the Examples herein used the same lyophilization chamber, the same supplies (vials, lyophilization stoppers, aluminum stoppers, inert atmospheres, etc.), and the same analytical methods (temperature probes in internal vials, analytical tests, etc.). In some of the mixtures that were treated, temperature and vacuum conditions were varied somewhat, depending on the percentage FDP that was present in the mixture.

The following parameters describes lyophilization of a liquid containing 10% FDP, measured as the weight of the octahydrate salt. This was a very conservative approach, evaluated during an early stage of the research. In addition, these times were high, because of the relatively deep quantity of solution required for 10 or 20% FDP in aqueous solution. Subsequent tests involving 30 or 40% solutions of FDP used shallower liquid depths, and were tested more aggressively, using substantially shorter times.

The shelves were initially cooled to -45°C in 56 minutes, and then cooled further, to -67°C, over five hours. Vacuum was initiated, and when the pressure reached 250 millitorr (mtorr), the shelf temperature was raised to -40°C and maintained there for 162 hours. The shelf temperature was maintained between -20°C and -40°C for an additional 240 hours, and then raised to 10°C for 15 hours. The temperature was then adjusted to 20°C for 5 hours. Pressure was maintained between 50 and 100 mtorr throughout the cycle.

After this drying cycle was completed, the vacuum was released, and nitrogen or argon was bled into the lyophilization chamber. The vials were sealed inside the lyophilizer under an atmosphere of inert gas, at atmospheric pressure. Aluminum overseals were placed on the vials and crimped into place. This generated a dried cake mass which contained about 14% water, by weight.

The stability of this product was determined from real time stability studies, using high performance liquid chromatography (HPLC) analysis on a "CarboPac PA1" column (Dionex Corp. Sunnyvale, CA) using a Beckman pump and controller (Beckman Company, Fullerton, CA) with a Waters model 464 Pulsed Amperometric Detector (Waters Company, Milford, MA). The shelf life was found to be at least 14 months when the product was stored at room temperature (25°C or lower).

It should be noted that the preparations described herein were not completely sterile. Although the liquids were sterile-filtered after the FDP power was dissolved in water, the powders and the vials were not handled under completely sterile conditions, in the lab-scale research which led to this invention. Accordingly, the excellent levels of chemical stability disclosed herein should be regarded as having an extra margin of safety; these stability levels were achieved despite a significant risk of biological contamination due to the non-sterile lab-scale handling procedures.

### EXAMPLE 4: LYOPHILIZATION OF 20% FDP SOLUTION

FDP (400 g of bulk powder, sodium salt including octahydrate water) was dissolved in 2 liters of water. The solution was pumped through a Gelman Acrodisc 0.2 micron filter, as above, and filled into the vials (about 25 mL of solution/vial). The vials were loaded onto the lyophilizer shelves at 0°C. The shelves were cooled to -40°C in ten hours and maintained at -40°C over 1.75 hours. The shelf temperature was then raised to -18°C over 4.75 hours and then lowered to -64°C in two hours. Vacuum was then initiated. The shelf temperature was raised to -30°C and maintained there for nine and a half days. The shelf temperature was then raised to 20°C over 8.5 hours, and then maintained there for 19.5 hours. The pressure was maintained between 4 and 70 mtorr throughout the cycle. The vacuum was released, and the vials were sealed at atmospheric pressure.

This cake had a residual moisture content of 11.9%. HPLC analysis of a sample that had been stored at room temperature for 343 days indicated 0.8% total impurities.

### EXAMPLE 5: LYOPHILIZATION OF 30% FDP SOLUTION

FDP (420 g of bulk powder) was dissolved in 1.4 liters of water (1.4 L), and pumped through the filter. 17 mL of solution was loaded into each vial. The solution depth was about 14 mm, measured next to the wall; depths in the middle of the vial were shallower. The vials were loaded onto the lyophilizer shelves at -55°C. The shelves were cooled to -67°C and vacuum was initiated when the product temperature reached -65°C. When the pressure reached 16 mtorr the shelf temperature was raised to -15°C and maintained there for 36 hours. The shelf temperature was then raised to 20°C over 30 minutes and then maintained there for three and a half hours. The pressure was below 100 mtorr throughout the cycle. The vacuum was released, and the vials were sealed at atmospheric pressure.

This produced a cake with a residual moisture content of 14.5%. Analysis of a sample that had been stored at room temperature for 201 days indicated 0.7% total impurities.

### EXAMPLE 6: LYOPHILIZATION OF 40% FDP SOLUTION, WITHOUT TBA

FDP (420 g) was dissolved in water (1.05 L) and pumped through the filter. 12.5 mL was loaded into each vial (depth next to wall was about 11 mm), and the vials were loaded onto the lyophilizer shelves at -57°C. The shelves were cooled to -70°C and vacuum was initiated when the product temperature reached -68°C. The shelves were allowed to warm to -45°C during vacuum pull-down, and when the pressure reached 71 mtorr the shelf temperature was raised to -10°C over 1.25 hours and maintained there for 20 hours. The shelf temperature was then raised to 23°C over 30 minutes and then maintained there for 19 hours. The pressure was below 100 mtorr throughout the cycle. The vacuum was released and the vials were sealed at atmospheric pressure.

The resulting cake collapsed, and was a pale yellow color. It was regarded as unacceptable, and was not analyzed for impurities. Instead, attention was turned to testing TBA as an agent to promote proper lyophilization, as described below.

### EXAMPLE 7: LYOPHILIZATION OF 40% FDP SOLUTION WITH 10% TBA

Tertiary butyl alcohol (105 mL) was mixed with 600 mL of water. FDP (420 g) was dissolved in the mixture, then enough water was added to this mixture to give a final volume of 1050 mL. In other tests, TBA was added to the water after the FDP had been added; the sequence of mixing did not make any apparent difference.

The solution was pumped through the filter, and 12.5 mL aliquots were loaded into the 100 mL vials. The vials were loaded onto the lyophilizer shelves at 2.5°C. The shelves were cooled to -67°C over 110 minutes and vacuum was initiated when the product temperature reached -60°C. The shelves were allowed to warm to -45°C during vacuum pull-down and maintained for one-half hour. The shelf temperature was raised to -10°C over 68 minutes and maintained there for 20 hours. The shelf temperature was then raised to 23°C over 30 minutes and then maintained there for 17 hours. The pressure was below 110 mtorr throughout the cycle. The vacuum was released, and the vials were sealed.

This produced a fragile cake with multiple cracks, which broke apart and produced a powder when shaken. Residual water content was 13.2%, and HPLC showed 0.1% total impurities after 15 days at room temperature.

The major apparent differences which resulted when TBA was used, in a number of tests, involved the structure and color of the cake. With a 100% aqueous formulation, resulting FDP cakes tend to be relatively dense and hard, and were sometimes off-white or slightly yellow. When TBA was used, the cakes were substantially lighter in color, approaching a pure, bright white. They are also less dense and more fragile, and sometimes broke into powders if shaken vigorously.

The preparations above, using 30% or 40% FDP and TBA in the aqueous mixture, produced partially lyophilized preparations of FDP which appeared to have very good quality. These were prepared only recently; accordingly, extended data on shelf life is not yet available, but initial results appear to be very good.

Except as noted above, these preparations, including some that were prepared without TBA and some that were prepared with TBA, are believed to be completely suitable for reconstitution and injection into humans who need emergency treatment for an ischemic or hypoxic crisis, or who need various other forms of medical treatment by physicians.

Thus, there has been shown and described a new and useful method for preparing partially lyophilized forms of FDP for injection into human patients. There have also been shown certain chemical compositions that result from this lyophilization method, and certain articles of manufacture which contain partially lyophilized FDP as a key component. Although the processing method, chemical compositions, and articles of manufacture described herein have all been exemplified for purposes of illustration and description by reference to certain specific embodiments, it will be apparent to those skilled in the art that various modifications, alterations, and equivalents of the illustrated examples are possible. Any such changes which derive directly from the teachings herein, and which do not depart from the spirit and scope of the invention, are deemed to be covered by this invention.

### REFERENCES

Angelos, M.G., et al, "Fructose-1,6-diphosptlate fails to limit early myocardial infarction size in a canine model," *Ann. Emerg. Med*. *22*: 171-177 (1993)
Eddy, L.J., et al, "Lack of a direct metabolic effect of fructose, 1,6-diphosphate in ischemic myocardium," *Am J Physiol 241*: H576-83 (1995)
Gregory, G.A., et al, "Fructose-1,6-bisphosphate reduces ATP loss from hypoxic astrocytes," *Brain Res 516*: 310-2 (1990)
Markov, A.K., et al, "Hemodynamic, electrocardiographic, and metabolic effects of fructose diphosphate on acute myocardial ischemia," *Am Heart J 100*: 639-46 (1980)
Markov, A.K., "Hemodynamics and metabolic effects of fructose 1-6 diphosphate in ischemia and shock--experimental and clinical observations," *Ann Emerg Med 15*: 1470-7 (1986)
Markov, A.K., et al, "Increasing survival of dogs subjected to hemorrhagic shock by administration of fructose 1-6 diphosphate," *Surgery 102*: 515-27 (1987)
Pasque, M.K., et a, "Metabolic intervention to affect myocardial recovery following isehemia," *Annals of Surgery 200*: 1-12 (1984)
Sano, W., et al, "Beneficial effect of fructose-1,6-bisphosphate on mitochondrial function during ischemia-reperfusion of rat liver," *Gastroenterology 108*: 1785-92 (1995)
Tortosa, A., et al, "Fructose-1,6-bisphosphate fails to ameliorate delayed neuronal death in the CA1 area after transient forebrain ischaemia in gerbils," *Neuropharmacology 32*: 1367-71 (1992)
Trimarchi, G.R., et al, "Effects of fructose-1,6-bisphosphate on brain polyamine biosynthesis in a model of transient cerebral ischemia," *Life Sci 54*: 1195-204 (1994)
Zhang, J.N., et al, "Protective effect of exogenous fructose-1,6-diphosphate in cardiogenic shock," *Cardiovasc Res 22*: 927-32 (1988)

## Claims

1. Use of fructose-1,6-diphosphate or a pharmacologically acceptable salt thereof in the manufacture of a medicament for use in a method for treating a human patient who is preparing to undergo circulatory bypass of the heart during surgery, the method comprising intravenously injecting into the patient a liquid formulation containing fructose-1,6-diphosphate or a salt thereof as a myocardial protective agent, wherein the fructose-1,6-diphosphate or salt thereof is intravenously injected into the patient, prior to commencement of circulatory bypass, under conditions which cause the fructose-1,6-diphosphate or salt thereof to permeate into heart muscle tissue while the heart is still beating, and wherein the fructose-1,6-diphosphate or salt thereof is injected into the patient in a therapeutically effective quantity which has been statistically shown to reduce ischemic heart stress during surgery.

2. Use according to claim 1, wherein the fructose-1,6-diphosphate or salt thereof is intravenously infused into the patient over a sustained period of time.

3. Use according to claim 1 or 2, wherein the intravenous injection of fructose-1,6-diphosphate or salt thereof commences at least about 10 minutes before commencement of circulatory bypass.

4. Use according to claim 1, 2 or 3, wherein fructose-1,6-diphosphate or salt thereof is intravenously injected into the patient at a dosage of at least about 100 milligrams of fructose-1,6-diphosphate or salt thereof per kilogram of patient body weight.

5. Use according to any one of claims 1 to 4 wherein intravenous injection of fructose-1,6-diphosphate or salt thereof prior to commencement of circulatory bypass is supplemented by addition of fructose-1,6-diphosphate or a pharmacologically acceptable salt thereof to a cardioplegia liquid that is circulated through the heart during circulatory bypass.

6. Use according to any one of claims 1 to 5 wherein the quantity of fructose-1,6-diphosphate or salt thereof which is injected into the patient is therapeutically effective in reducing creatine kinase release by heart tissue during and after surgery.

7. Use according to any one of claims 1 to 6 wherein the quantity of fructose-1,6-diphosphate or salt thereof which is injected into the patient is therapeutically effective in reducing aberrations in hemodynamic pumping performance by the patient's heart following surgery.

8. Use according to any one of claims 1 to 7 wherein the patient is being operated on to replace at least one segment of an occluded coronary artery.

9. Use of fructose-1,6-diphosphate or a pharmacologically acceptable salt thereof in the manufacture of a medicament for use in the emergency treatment of a patient, by intravenous injection of fructose-1,6-diphosphate or salt thereof into a human patient who appears to be suffering from a condition indicating a medical emergency, wherein the condition is selected from:
a. loss of blood;
b. abnormalities in pulse;
c. electrical shock;
d. symptoms of heart ischemia;
e. asphyxiation;
f. loss of consciousness;
g. mental disorientation which does not appear to be related to drugs or alcohol;
h. inability to control motor functions which does not appear to be related to drugs or alcohol; and,
i. apparent haemorrhagic shock,
and wherein the fructose-1,6-diphosphate or salt thereof is intravenously injected into the patient, prior to diagnosis of the patient's condition.

10. Use according to claim 9, wherein fructose-1,6-diphosphate or a pharmacologically acceptable salt thereof is injected into the patient in an initial bolus having a dosage in a range of 50 to 400 milligrams of fructose-1,6-diphosphate or salt thereof per kilogram of patient body weight.

11. Use according to claim 9 or 10, wherein an initial bolus injection of fructose-1,6-diphosphate or salt thereof is followed by intravenous infusion of fructose-1,6-diphosphate or a pharmacologically acceptable salt thereof at a dosage in a range of 1 to 5 milligrams of fructose-1,6-diphosphate or salt thereof per kilogram of patient body weight.
